# EUROPEAN PATENT APPLICATION

(11) **EP 3 081 627 A1**
(43) Date of publication of application: **19.10.2016**
(21) Application number: 13898961.1
(22) Date of filing: 12.12.2013
(51) Int. Cl.: C12M 1/00

(54) **WELL PLATE AND SUBJECT SELECTION DEVICE PROVIDED WITH WELL PLATE**

(71) Applicant: Yamaha Hatsudoki Kabushiki Kaisha, Iwata-shi, Shizuoka-ken 438-8501 (JP)
(72) Inventor: ITO, Saburo, Shizuoka-ken 438-8501 (JP)
(74) Representative: Müller-Boré & Partner Patentanwälte PartG mbB
(86) International application number: PCT/JP2013/007330
(87) International publication number: WO 2015/087369

(57) **Abstract**

Provided is a well plate that carries, in a carrying position, a subject retained in liquid, the well plate including an upper surface, a lower surface, and a plurality of recessed sections formed in the carrying position. Each of the plurality of recessed sections is opened on the upper surface side, has a shape recessed from the upper surface side to the lower surface side, and includes, in a cross section in an up-down direction, a bottom section in which a first surface having a zero curvature or a first curvature is formed and the subject is carried, and a side section in which a second surface having a second curvature larger than the first curvature and continuous to the first surface is formed.

## Description

### Technical Field

The present invention relates to a well plate that carries subjects such as cells and a subject selection device provided with the well plate.

### Background Art

In various fields, there have been proposed methods of selecting subjects according to sizes and external shapes (hereinafter sometimes simply referred to as shapes). Examples of the subjects to be selected include, as large subjects, tablets, capsules, and granulated granules and include, as small subjects, cells deriving from organisms used in the fields of biotechnology-related techniques and medicines. For example, if the cells are selected and the shapes thereof are aligned in this way, it is possible to reduce deviations of experiment conditions in various experiments performed using the cells. The cells after the selection can be used for high-throughput screening (HTS) and the like.

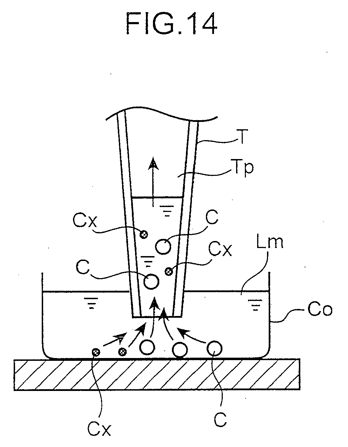

However, for example, when only cells having a shape suitable for an experiment are sucked and selected from a plurality of cells assuming various shapes, other impurities are sometimes simultaneously sucked during the selection. Fig. 14 is a schematic view for explaining a state in which impurities are simultaneously sucked. As shown in Fig. 14, when liquid Lm such as a cell culture solution is stored in a container Co opened in an upper part such as a dish and a plurality of cells C and impurities Cx are carried on the liquid Lm, if a suction force is generated in a tubular passage Tp of a suction chip T by a suction device (not shown in the figure) such as a suction pipet, the impurities Cx are sucked into the tubular passage Tp together with the cells C. As a result, only the cells C cannot be selected. Whereas one cell C should be sucked, a plurality of cells C are sometimes simultaneously sucked.

In view of such problems, Patent Literature 1 discloses a method of manufacturing a platen having desired thickness including a plurality of through-holes. The platen of Patent Literature 1 includes the plurality of through-holes and carries cells and the like in the through-holes to thereby perform selection of cells of a desired size and thereafter collects the cells with suction or the like.

However, the through-holes described in Patent Literature 1 carry the cells and the like on steep slopes formed in the four directions. Therefore, the carried cells and the like are easily deformed along the shape of the through-holes and the characteristics of the cells and the like sometimes change. The carried cells firmly fit in the through-holes and are sometimes damaged when being forcibly collected by suction or the like. Further, a plurality of cells sometimes fit in one through-hole. In such a case, the plurality of cells are not easily separated even if external force such as vibration is applied thereto. It is difficult to appropriately collect one cell.

### Citation List

### Patent Literature

Patent Literature 1: JP 2009-504161 A

### Summary of Invention

The present invention has been devised in view of such conventional problems and it is an object of the present invention to provide a well plate on which a carried subject is less easily deformed, even if a plurality of subjects are carried, the plurality of subjects can be easily separated by applying external force such as vibration thereto, and the subject can be collected without changing characteristics of the subject and without damaging the subject and a subject selection device provided with the well plate.

A well plate according to an aspect of the present invention is a well plate that carries, in a carrying position, a subject retained in liquid, the well plate including an upper surface, a lower surface, and a plurality of recessed sections formed in the carrying position, wherein each of the plurality of recessed sections is opened on the upper surface side, has a shape recessed from the upper surface side to the lower surface side, and includes, in a cross section in an up-down direction, a bottom section in which a first surface having a zero curvature or a first curvature is formed and the subject is carried, and a side section in which a second surface having a second curvature larger than the first curvature and continuous to the first surface is formed.

Objects, features, and advantages of the present invention are made clearer by the following detailed explanation and the accompanying drawings.

### Brief Description of Drawings

[Fig. 1] Fig. 1 is a perspective view of a well plate of a first embodiment of the present invention.
[Fig. 2] Fig. 2 is a sectional view of a recessed section of the first embodiment of the present invention.
[Fig. 3] Fig. 3 is a top view of the recessed section of the first embodiment of the present invention.
[Fig. 4] Fig. 4 is a sectional view of the recessed section of the first embodiment of the present invention.
[Fig. 5] Fig. 5 is a sectional view of the recessed section of the first embodiment of the present invention.
[Fig. 6] Fig. 6 is a schematic view of end face shapes of side sections in cross sectional positions (1) to (3) in Fig. 2, wherein Fig. 6A is a schematic view of the end face shape of the side section in the cross sectional position (1) in Fig. 2, Fig. 6B is a schematic view of the end face shape of the side section in the cross sectional position (2) in Fig. 2, and Fig. 6C is a schematic view of the end face shape of the side section in the cross sectional position (3) in Fig. 2.
[Fig. 7] Fig. 7 is a sectional view of a recessed section of a second embodiment of the present invention.
[Fig. 8] Fig. 8 is a perspective view of a well plate of a third embodiment of the present invention.
[Fig. 9] Fig. 9 is a sectional view of a recessed section of the third embodiment of the present invention.
[Fig. 10] Fig. 10 is a top view of the recessed section of the third embodiment of the present invention.
[Fig. 11] Fig. 11 is a schematic view of end face shapes of side sections in cross sectional positions (4) to (6) in Fig. 9, wherein Fig. 11A is a schematic view of the end face shape of the side section in the cross sectional position (4) in Fig. 9, Fig. 11B is a schematic view of the end face shape of the side section in the cross sectional position (5) in Fig. 9, and Fig. 11C is a schematic view of the end face shape of the side section in the cross sectional position (6) in Fig. 9.
[Fig. 12] Fig. 12 is a schematic view for explaining the configuration of a subject selection device of a fourth embodiment of the present invention.
[Fig. 13] Fig. 13 is a sectional view for explaining a well plate of a modification of the first embodiment of the present invention.
[Fig. 14] Fig. 14 is a schematic view for explaining a state in which impurities are simultaneously sucked.

### Description of Embodiments

### <Well plate>

### (First Embodiment)

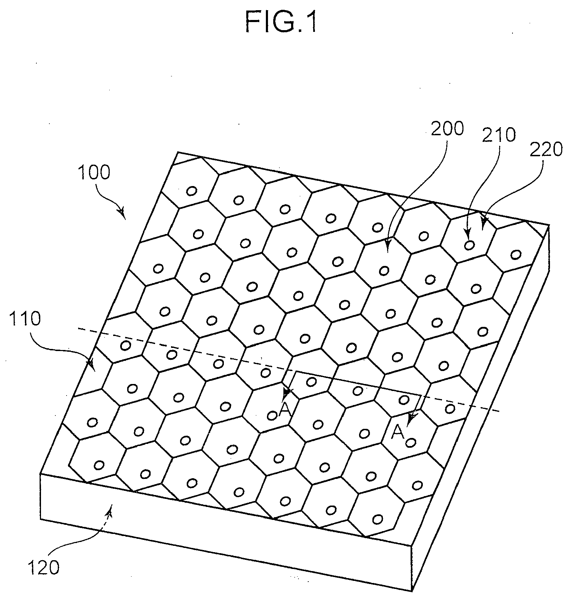

A well plate of a first embodiment of the present invention is explained in detail with reference to the drawings. Fig. 1 is a perspective view of a well plate 100 of this embodiment.

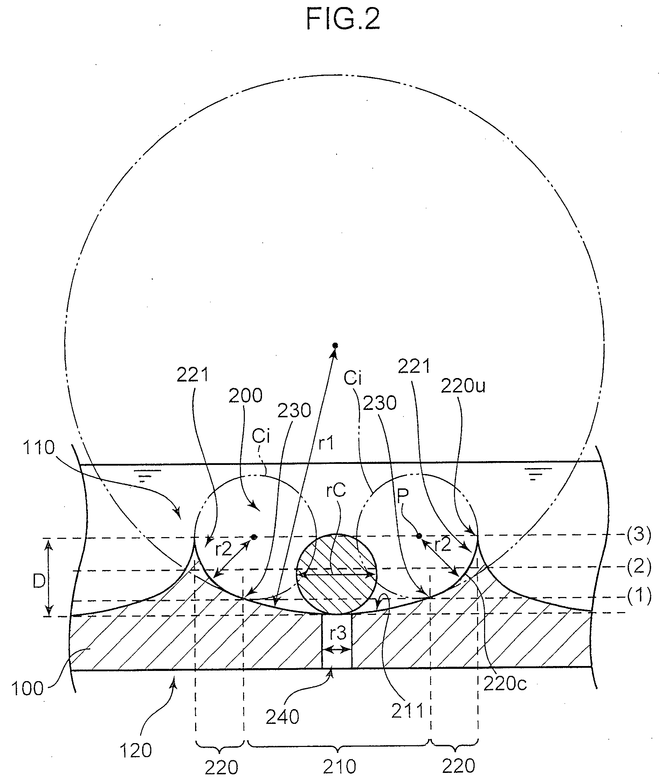

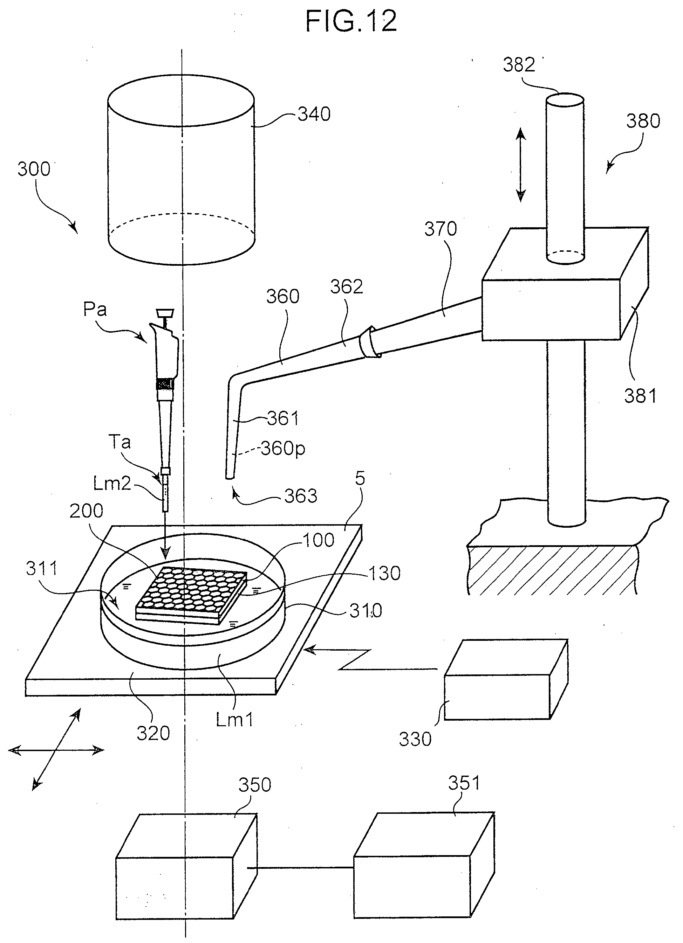

A well plate 100 of this embodiment is a member for carrying cell aggregates C (spheroids, subjects, see Fig. 2) retained in a cell culture solution Lm1 (liquid). For example, the well plate 100 is immersed in a container such as a petri dish 310, in which the cell culture solution Lm1 is stored, and used (see Fig. 12). The carried cell aggregates C can be observed by, for example, an image pickup device 350 such as a phase contrast microscope provided outside (observing means, see Fig. 12). The well plate 100 includes an upper surface 110 and a lower surface 120.

The shape of the well plate 100 is not particularly limited but is desirably a flat shape because it is easy to focus the phase contrast microscope, for example, when the carried cell aggregates C are observed from below by the image pickup device 350 (see Fig. 12) such as the phase contrast microscope provided outside. As the size of the well plate 100, it is sufficient that width is smaller than opening width of the dish 310 and height is small compared with housing depth of the petri dish 310 because the well plate 100 needs to be immersed in the cell culture solution Lm1 stored in the petri dish 310. The well plate 100 of this embodiment includes a 15 mm square flat rectangular parallelepiped shape having height of 0.15 mm.

The material of the well plate 100 is not particularly limited and is desirably a translucent material because it is possible to easily check a state of the cell aggregates C. The translucent material is not particularly limited. However, examples of the translucent material include thermoplastic resin, thermosetting resin, and photosetting resin. More specifically, examples of the translucent material include polyethylene resin; polyethylene naphthalate resin; polypropylene resin; polyimide resin; polyvinyl chloride resin; cycloolefin copolymer; norbornene containing resin; polyether sulfone resin; polyethylene naphthalate resin; cellophane; aromatic polyamide resin; (meta) acrylic resin such as poly(meta) methyl acrylate; styrene resin such as polystyrene and styrene-acrylonitrile copolymer; polycarbonate resin; polyester resin; phenoxy resin; butyral resin; polyvinyl alcohol; cellulose-based resin such as ethyl cellulose, cellulose acetate, cellulose acetate butyrate; epoxy resin; phenol resin; silicone resin; and polylactic acid. Besides, examples of the material of the well plate 100 include an inorganic material, for example, metal alkoxide, ceramic precursor polymer, a solution obtained by subjecting a solution containing metal alkoxide to hydrolytic polymerization with a sol-gel method, or an inorganic material obtained by solidifying a combination of these, for example, an inorganic material containing siloxane bonding (polydimethylsiloxane or the like), and glass. The well plate 100 of this embodiment is made of acrylic.

On the well plate 100, a plurality of recessed sections 200 recessed from the upper surface 110 side to the lower surface 120 side are formed in carrying positions of the cell aggregates C. Therefore, in the well plate 100, for example, by dripping the cell culture solution Lm1 including the cell aggregates C from above with a suction pipet attached with a suction chip, it is possible to drop the cell aggregates C and carry the cell aggregates C on the respective recessed sections 200. Whereas one cell aggregate C should be carried on the recessed section 200, when the cell culture solution Lm1 including the cell aggregates C is dripped from above in this way, in some case, a plurality of cell aggregates C are carried on one recessed section 200 or impurities other than the cell aggregates C are carried on one recessed section 200 together with the cell aggregates C. The recessed sections 200 of this embodiment have a shape described below. Therefore, even in such cases, by applying external force such as vibration, it is possible to separate the plurality of cell aggregates C and the impurities and carry only one cell aggregate C on the recessed section 200. When the cell aggregate C is sucked from one recessed section 200 and selected, the cell aggregates C carried on the other recessed sections 200 are not simultaneously sucked.

Fig. 2 is a sectional view of the recessed sections 200 of this embodiment and is a sectional view in a position indicated by A-A in Fig. 1. Each of the plurality of recessed sections 200 has a substantially cup shape opened on the upper surface 110 side and recessed from the upper surface 110 side to the lower surface 120 side. More specifically, each of the plurality of recessed sections 200 of this embodiment includes, in a cross section in the up-down direction, a bottom section 210 in which a first surface 211 having a first curvature is formed and a side section 220 in which a second surface 221 having a second curvature and continuous to the first surface 211 is formed. The bottom section 210 and the side section 220 are smoothly continuous in a continuous section 230.

The bottom section 210 is a part in which the cell aggregate C is mainly carried. The bottom section 210 includes the first surface 211, which is a carrying surface on which the cell aggregate C is carried. The first surface 211 is a curved surface having the first curvature. The periphery of the first surface 211 is connected to the second surface 221 of the side section 220 via the continuous section 230. The first curvature is not particularly limited and only has to be a curvature for enabling the cell aggregate C to be carried without being deformed. Depending on the size of the recessed section 200, for example, when a maximum diameter of an opening formed on the upper surface 110 side of the recessed section 200 is 0.5 mm, such a curvature is larger than zero and equal to or smaller than 1.75 (mm⁻¹). In this case, a curvature radius r1 is equal to or larger than 0.57 mm and does not include infinity (∞). In this embodiment, in the recessed section 200, the maximum diameter of the opening of which is 0.37 mm, the bottom section 210 is illustrated in which the first surface 211, the first curvature of which is 4.55 (mm⁻¹) and the curvature radius r1 of which is 0.22 mm, is formed. The first surface 211 having the first curvature is a curved surface. However, since the curvature is large, the first surface 211 is formed relatively flat. Therefore, the cell aggregate C is stably carried without being deformed in the bottom section 210 in which the first surface 211 having the first curvature is formed.

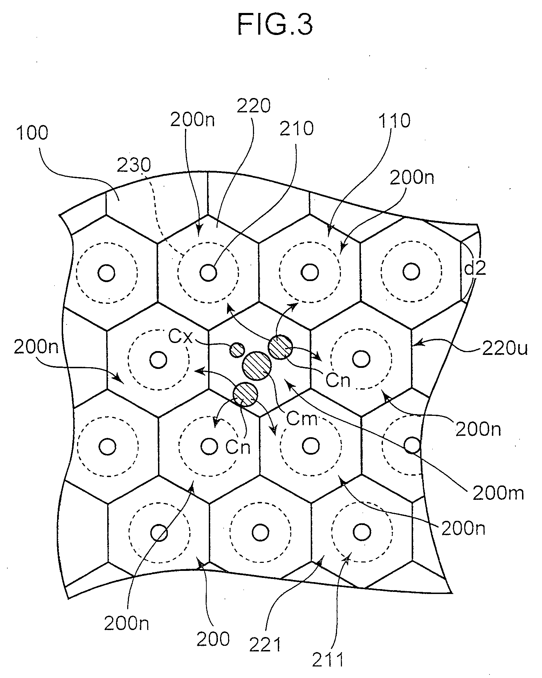

In the center of the bottom section 210, a through-hole 240 piercing through the well plate 100 from the upper surface 110 side to the lower surface 120 side is formed. A diameter r3 of the through-hole 240 is not particularly limited and only has to be smaller than a minimum diameter rC of the cell aggregate C that should be carried. As explained below, the cell aggregate C has a substantially spherical shape. The minimum diameter rC of the cell aggregate C is approximately 0.05 to 0.1 mm. Therefore, the diameter r3 of the through-hole 240 only has to be, for example, 0.008 to 0.05 mm. The number of through-holes 240 is not particularly limited and may be one and may be plural. Further, the depth of the through-hole 240 is not particularly limited and is set as appropriate taking into account the strength and the like of the well plate 100. In this embodiment, it is illustrated that one columnar through-hole 240, the diameter r3 of which is 0.04 mm and the depth of which is 0.05 mm, is formed in the center of the bottom section 210. Since such a through-hole 240 is formed in the bottom section 210, even when an impurity Cx (see Fig. 3) having a diameter smaller than the cell aggregate C drops to the recessed section 200, such an impurity Cx passes through the through-hole 240 and drops without being carried by the bottom section 210. Since the diameter r3 of the through-hole 240 is smaller than the diameter rC of the cell aggregate C, the cell aggregate C less easily fits in the through-hole 240 and is less easily deformed.

The side section 220 includes the second surface 221, which is a curved surface having the second curvature, and includes a lower end 220d smoothly continuous to the periphery of the bottom section 210 via the continuous section 230 and an upper end 220u including a peripheral edge. The second curvature is larger than the first curvature. As such curvatures, depending on the size of the recessed section 200, for example, when the maximum diameter of the opening of the recessed section 200 formed on the upper surface 110 side is 0.5 mm, the curvatures are equal to or larger than 6.66 (mm⁻¹) and equal to or smaller than 20 (mm⁻¹). In this case, a curvature radius r2 is equal to or larger than 0.05 mm and equal to or smaller than 0.15 mm. In this embodiment, in the recessed section 200, the maximum diameter of the opening of which is 0.37 mm, the side section 220 is illustrated in which the second surface 221, the second curvature of which is 7.69 (mm⁻¹) and the curvature radius r2 of which is 0.13 mm, is formed. In the well plate 100, since the second surface 221 having such a second curvature is formed in the side sections 220 of the respective recessed sections 200, for example, even when the cell aggregate C drops to the side section 220 from the above, the cell aggregate C drops to roll along the second surface 221 and is carried by the bottom section 210.

When a circumferential portion of a curvature circle Ci having the second curvature is set in contact with the second surface 221, the position of the upper end of the second surface 221 (the upper end 220u of the side section 220) is set to be equal to or lower than the horizontal position of a center P of the curvature circle Ci. In the side section 220 of the recessed section 200 of this embodiment, when the circumferential portion of the curvature circle Ci having the second curvature (9.09 (mm⁻¹)) is set in contact with the second surface 221, the position of the upper end of the second surface 221 is designed to be a horizontal position same as the center P of the curvature circle Ci. Therefore, the second surface 221 is formed such that the vicinity of the upper end faces substantially the vertical direction and is not formed in a shape bending in the center direction of the recessed section 200. As a result, the cell aggregate C can be separated from the recessed section 200 when the vibration is applied to the cell aggregate C by a vibration generating device (vibration generating means, see Fig. 12) explained below.

Depending on the second curvature, vertical height D of the upper end 220u of the side section 220 with respect to the deepest section of the bottom section 210 is desirably designed to be 0.06 to 0.5 mm. If the vertical height D of the upper end 220u of the side section 220 is in such a range, when external force such as vibration is applied when a plurality of cell aggregates C are carried on the recessed sections 200 explained below, the cell aggregates C are separated and only one cell aggregate C is carried on the bottom section 210. When a suction pipet attached with a suction chip is inserted from above and a suction force is generated in order to suck only the cell aggregate C of one recessed section 200 when the cell aggregates C are carried in the respective recessed sections 200 adjacent to each other, the cell aggregates C of the neighboring recessed sections 200 are not simultaneously sucked and only the cell aggregate C of one recessed section 200 is sucked. In this embodiment, the recessed section 200, the vertical height D of which is 0.1 mm, is illustrated. Action and effects of these sections are more specifically explained with reference to Fig. 3 to Fig. 5.

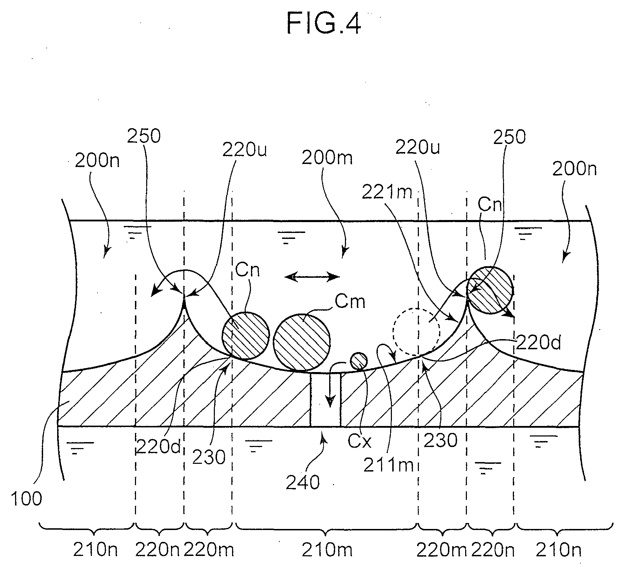

First, an effect of appropriate separation of a plurality of cell aggregates is explained. Fig. 3 is a top view of the recessed section 200 of this embodiment. Fig. 4 is a sectional view of the recessed section 200 of this embodiment. In the respective plurality of recessed sections 200, the peripheral edges of the upper ends 220u of the side sections 220 are connected. In one of the plurality of recessed sections 200, a plurality of cell aggregates and impurities are carried. In this embodiment, as an example, three cell aggregates (a cell aggregate Cm and cell aggregates Cn) and one impurity Cx are carried on one recessed section 200m (a first recessed section). The recessed section 200m includes a bottom section 210m in which a first surface 211m having the first curvature is formed and a side section 220m in which a second surface 221m having the second curvature is formed. The cell aggregate Cm is carried on substantially the center of the bottom section 210m. The cell aggregates Cn are carried around the cell aggregate Cm. The cell aggregates Cn are carried on the bottom section 210m or in the vicinity of the boundary between the bottom section 210m and the side section 220m. The peripheral edge of the side section 220m of the recessed section 200m and the peripheral edges of side sections 220n of recessed sections 200n are connected. A pointed peak section 250 is formed. When the vibration generating device (not shown in the figure) provided outside is driven to apply vibration to the well plate 100 in such a state, stress due to the vibration is applied to the cell aggregate Cm and the cell aggregates Cn. Among the cell aggregate Cm and the cell aggregates Cn, the cell aggregate Cm is not moved much because the cell aggregate Cm is carried substantially in the center of the bottom section 210m on the relatively flat first surface 211m. However, the cell aggregates Cn carried around the cell aggregate Cm climb over the side section 220m and are separated to the neighboring recessed sections 200n (second recessed sections adjacent to the first recessed section). In this case, since the peak section 250 is pointed, when the cell aggregates Cn climb over the side section 220m and approach the peak section 250, if the cell aggregates Cn climb over the peak section 250 once without being held in the peak section 250, the cell aggregates Cn drop to bottom sections 210n along the side sections 220n of the neighboring recessed sections 200n. The impurity Cx drops from the through-hole 240 formed in the bottom section 210 or separated to the outside of the recessed section 200m by the stress applied by the vibration. Note that the cell aggregate Cm carried substantially in the center of the bottom section 210m sometimes slightly moves with the stress applied by the vibration. However, a moving distance of the cell aggregate Cm is in a range in which the cell aggregate Cm does not move out of the bottom section 220m in which the first surface 221m is formed. Even if the cell aggregate Cm moves out of the bottom section 220m, the cell aggregate Cm only approaches a side section 230m. Therefore, the cell aggregate Cm is not moved to climb over the side section 220m. Even if the cell aggregate Cm moves to the side section 220m, the cell aggregate Cm drops in the direction of the bottom section 210m along the side section 220m and is carried on the bottom section 210m again. Note that the cell aggregates may be separated by tilting the well plate 200 to the front and the back and to the left and the right besides applying the external force such as the vibration.

As shown in Fig. 3, the shape of the opening in top view in each of the plurality of recessed sections 200 of this embodiment is a regular hexagon. The plurality of recessed sections 200 are arrayed in a honeycomb shape. Therefore, the cell aggregates Cn are easily separated from the recessed section 200m to the recessed sections 200n adjacent in six directions when vibration is applied to the cell aggregates Cn. Since the plurality of recessed sections 200 are densely arrayed in the honeycomb shape, the number of recessed sections 200 that can be formed in one well plate 100 increases and area efficiency is high. Note that, when the cell aggregate Cm and the cell aggregates Cn are carried on the recessed section 200m, vibration conditions necessary for separating the cell aggregates n and carrying only the cell aggregate Cm in the recessed section 200m are set as appropriate on the basis of the shape and the mass of the cell aggregates, the viscosity and the temperature of a cell culture solution, the shape of the recessed sections, and the like. As an example, in the well plate 100 of this embodiment, as explained above, the first curvature is 4.55 (mm⁻¹), the second curvature is 6.66 (mm⁻¹), the depth of the recessed section 200 (the vertical height D from the deepest section of the bottom section 210 to the upper end 220u of the side section 220) is 0.1 mm, and the maximum diameter of the opening of the recessed section 200 is 0.37 mm. The substantially spherical cell aggregates C, the diameter rC of which is 0.1 mm, are carried on the bottom section 210 (see Fig. 2). It is assumed that the specific gravity, the viscosity, and the like of the cell culture solution Lm1 are the same degrees as those of water. In this case, when a vibration frequency is set to 0 to 200 rpm when the vibration is applied by the vibration generating device or an inclination angle is set to approximately ±5 to 10° when the well plate 200 is tilted. Consequently, it is possible to separate the cell aggregates Cn carried on the recessed section 200m and carry only the cell aggregate Cm on the recessed section 200m.

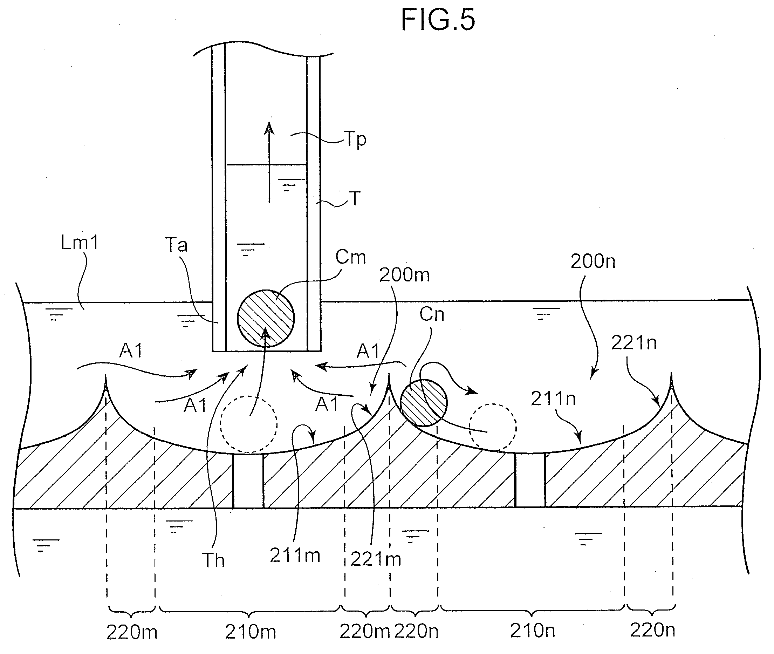

An effect of appropriate suction of only one cell aggregate is explained. Fig. 5 is a sectional view of the recessed section of this embodiment. A cell aggregate is carried in each of the plurality of recessed sections. In this embodiment, as an example, one cell aggregate Cm is carried on the bottom section 210m of one recessed section 200m, one cell aggregate Cn is carried on the bottom section 210n of the neighboring recessed section 200n of the recessed section 200m, and only the cell aggregate Cm is sucked.

First, when the suction chip T of a suction pipet (not shown in the figure) is inserted from above the recessed section 200m to perform suction, the cell aggregate Cm to be sucked carried on the recessed section 200m and the cell culture solution Lm1 are sucked into the tubular passage Tp of the suction chip T. At this point, a liquid flow A1 occurs. The cell aggregate Cn carried on the recessed section 200n is sometimes moved by the liquid flow A1. However, in the respective recessed sections of this embodiment (e.g.; the recessed section 200n), the bottom section 210n in which a first surface 211n having the first curvature is formed and the side section 220n in which a second surface 221n having the second curvature larger than the first curvature is formed are formed. Therefore, the cell aggregate Cn is not moved by the liquid flow A1 to climb over the side section 220n. The cell aggregate Cn drops along the side section 220n and is careered in the bottom section 210n again. As a result, only the cell aggregate Cm carried on the recessed section 200m, into which the suction chip T is inserted, is sucked. Note that, when one cell aggregate C (the cell aggregate Cm and the cell aggregate Cn) is carried on each of the recessed sections 200 (the recessed section 200m and the recessed section 200n) adjacent to each other, suction conditions necessary for sucking only the cell aggregate Cm carried on the recessed section 200m without sucking the cell aggregate Cn carried on the neighboring recessed section 200n are set as appropriate on the basis of the shape and the mass of the cell aggregates, the viscosity and the temperature of the cell culture solution, the shape of the recessed sections, and the like. As an example, in the well plate 100 of this embodiment, as explained above, the first curvature is 4.55 (mm⁻¹), the second curvature is 6.66 (mm⁻¹), the depth of the recessed section 200 (the vertical height D from the deepest section of the bottom section 210 to the upper end 220u of the side section 220) is 0.1 mm, and the maximum diameter of the opening of the recessed section 200 is 0.37 mm The substantially spherical cell aggregates C, the diameter rC of which is 0.1 mm, are carried on the bottom section 210 (see Fig. 2). It is assumed that the specific gravity, the viscosity, and the like of the cell culture solution Lm1 are the same degrees as those of water. In this case, when the suction pipet (not shown in the figure) attached with the suction chip T is inserted from right above the recessed section 200m, a suction port Th is disposed 0.15 mm above the cell aggregate Cm, and suction is performed by 0.4 µL at speed of 0.8 µL/sec, only the cell aggregate Cm carried on the recessed section 200m is sucked and the cell aggregate Cn carried on the recessed section 200n is not sucked.

Referring back to Fig. 2, the continuous section 230 is a part that smoothly connect the bottom section 210 and the side section 220 and is formed in a place where the bottom section 210 and the side section 220 are continuous. The shape of the continuous section 230 is not particularly limited and is regulated by the outer peripheral shape of the first surface 211 of the bottom section 210. Specifically, when the outer peripheral shape of the first surface 211 of the bottom section 210 in top view is a circular shape (see Fig. 3), the continuous section 230 has the same circular shape in top view.

Referring to Fig. 4 again, an effect achieved by the smooth continuation of the bottom section 210 and the side section 220 by the continuous section 230 is explained. First, when a plurality of cell aggregates (the cell aggregate Cm and the cell aggregates Cn) are carried on the bottom section 210m and the cell aggregates Cn are separated to the recessed sections 200n leaving only the cell aggregate Cm in the recessed section 200m, if the bottom section 210m and the side sections 220m are not smoothly connected and a portion forming a corner is present, when external force (external energy) such as vibration is applied to the well plate 100, the cell aggregates Cn that receive the external energy and move from the bottom section 210m to the side section 220m sometimes collide with the portion of the corner. In this case, in some case, energy (internal energy) of the cell aggregates Cn obtained by the vibration and retained on the inside is lost, the cell aggregates Cn cannot climb over the side sections 220m, and are not separated from the recessed section 200m to the recessed sections 200n. However, since the bottom section 210 and the side section 220 are smoothly connected by the continuous section 230 of this embodiment, the cell aggregates Cn are easily moved from the bottom section 210m to the side section 220m and the internal energy is not lost and is used to climb over the side section 220. As a result, the cell aggregates Cn are easily separated from the recessed section 200m to the recessed sections 200n leaving only the cell aggregate Cm.

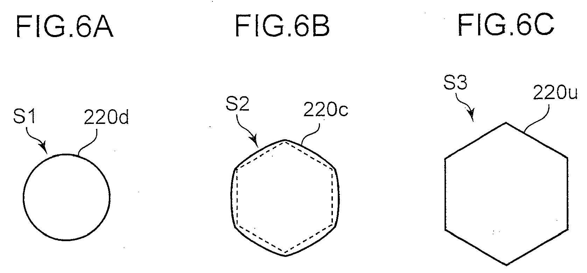

Note that, as explained above, the shape of the opening in top view in each of the plurality of recessed sections 200 of this embodiment is the regular hexagon (see Fig. 3). That is, the shape of the upper end 220u of the side section 220 is the regular hexagon in top view. On the other hand, since the lower end 220d of the side section 220 is smoothly connected to the bottom section 210 by the continuous section 230, the shape of the lower end 220d is the same as the outer peripheral shape of the bottom section 210 (e.g., a circular shape). Therefore, the shape of the side section 220 in top view on the lower end 220d side is the same as the outer peripheral shape of the bottom section 210 in top view. The shape of the side section 220 in top view on the upper end 220u side is a shape continuously deformed from the lower end 220d side to the upper end 220u side to be formed in the regular hexagon in top view. This is more specifically explained with reference to Fig. 6. Fig. 6 is a schematic view of end face shapes of the side section 220 in cross sectional positions (1) to (3) in Fig. 2. Fig. 6A is a schematic view of the end face shape of the side section 220 in the cross sectional position (1) in Fig. 2. Fig. 6B is a schematic view of the end face shape of the side section 220 in the cross sectional position (2) in Fig. 2. Fig. 6C is a schematic view of the end face shape of the side section 220 in the cross sectional position (3) in Fig. 2. As shown in Fig. 6A, in Fig. 2, a shape S1 of the lower end 220d of the side section 220 in the cross sectional position (1) is the same as the outer peripheral shape of the bottom section 210. In this embodiment, it is illustrated that the outer peripheral shape of the bottom section 210 is a circular shape and the end face shape of the lower end 220d of the side section 220 is also a circular shape. On the other hand, as shown in Fig. 6B, in Fig. 2, the shape of a center section 220c of the side section 220 in the cross sectional position (2) is a shape similar to the regular hexagon. However, sides connecting vertexes are deformed into a slightly rounded shape. As shown in Fig. 6C, in Fig. 2, the shape of the upper end 220u of the side section 220 in the cross sectional position (3) is the regular hexagon. In this way, the shape of the side section 220 is continuously deformed from the lower end 220d side to the upper end 220u side in top view. Therefore, when the cell aggregate C moves from the bottom section 210 climbing over the side section 220 when the external energy such as the vibration is applied thereto (in particular, near the continuous section 230 where the cell aggregate C approaches the side section 220 from the bottom section 210), internal energy is less easily lost. The cell aggregate C is easily separated climbing over the side section 220.

As explained above, with the well plate 100 of this embodiment, the first surface 211 of the bottom section 210 of the recessed section 200 has the first curvature smaller than the second curvature and is relatively flat. Therefore, the cell aggregate C is stably carried on such a relatively flat bottom section 210 and the shape of the cell aggregate C is not excessively deformed. As explained above with reference to Fig. 4, if the plurality of cell aggregates (the cell aggregate Cm and the cell aggregates Cn) are carried on the bottom section 210m or the impurity Cx is carried on the bottom section 210m, for example, by applying the external force such as the vibration, the cell aggregate C is easily separated to the neighboring recessed sections 200n from the bottom section 210m in which the first surface 211 m having the first curvature is formed through the side section 220m in which the second surface 221m having the larger second curvature is formed. As a result, only the cell aggregate Cm is carried on the recessed section 200m. As explained above with reference to Fig. 5, the second curvature of the second surface 221n formed in the side section 220n is larger than the first curvature of the first surface 211n formed in the bottom section 210n. Therefore, even if a liquid flow slightly occurs around the cell aggregate C carried on the bottom section 210n, the cell aggregate C is not moved climbing over the side section 220 by the liquid flow. Specifically, for example, even if a liquid flow occurs in the cell culture solution Lm1 when a distal end portion Ta including the suction port Th of the suction chip T is inserted from right above the recessed section 200m in order to suck the cell aggregate Cm carried on the neighboring recessed section 200m, the cell aggregates Cn carried on the bottom sections 210n of the recessed sections 200n are not moved to climb over the side section 220n by the liquid flow of this degree. Even if a suction force is generated in the tubular passage Tp of the suction chip T to suck the cell aggregate Cm in this state, the cell aggregates Cn carried on the recessed sections 200n are less easily affected by the liquid flow. As a result, with the well plate 100 of this embodiment, when the cell aggregate Cm to be sucked carried on the recessed section 200m is sucked, the cell aggregates Cn carried on the neighboring recessed sections 200n are not simultaneously sucked. Only the cell aggregate Cm to be sucked can be easily sucked and selected.

### (Second Embodiment)

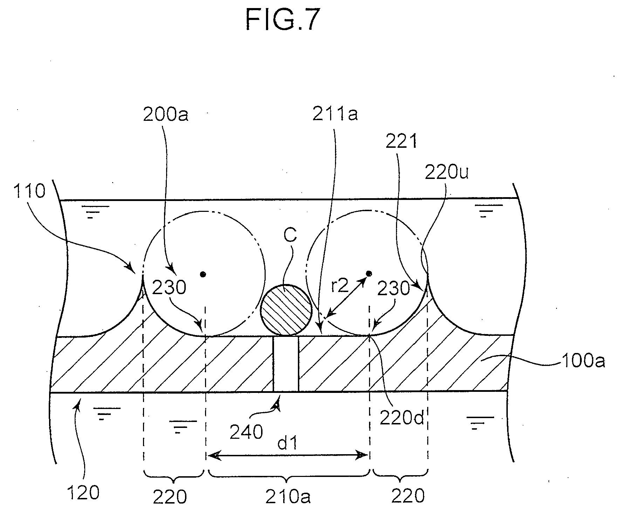

A well plate 100a of a second embodiment of the present invention is explained in detail below with reference to the drawings. Fig. 7 is a sectional view of the recessed section 200a of this embodiment. The well plate 100a of this embodiment has a configuration same as the configuration of the well plate 100 (see Fig. 2) explained above in the first embodiment except that the shape of a bottom section 210a of the recessed section 200a is different. Therefore, redundant components are denoted by the same reference numerals and signs and explanation of the components is omitted as appropriate.

Each of a plurality of recessed sections 200a of this embodiment has a substantially cup shape opened on the upper surface 110 side and recessed from the upper surface 110 side to the lower surface 120 side. More specifically, each of the plurality of recessed sections 200a of this embodiment includes, in a cross section in the up-down direction, a bottom section 210a in which a first surface 211 a having a zero curvature is formed and the side section 220 in which the second surface 221 having the second curvature and continuous to the first surface 211a is formed. The bottom section 210a and the side section 220 are smoothly continuous in the continuous section 230.

The bottom section 210a is a part in which the cell aggregate C is mainly carried and includes the first surface 211 a, which is a carrying surface. The first surface 211 a is a flat surface having a zero curvature. Since the first surface 211 a is the flat surface having the zero curvature in this way, the cell aggregate C is not deformed in the bottom section 210a and is more stably carried.

In the center of the bottom section 210a, the through-hole 240 piercing through the well plate 100a from the upper surface 110 side to the lower surface 120 side is formed. The dimension, the action, and the like of the through-hole 240 are the same as the dimension, the action, and the like explained above in the first embodiment. Therefore, explanation thereof is omitted.

Width d1 in the horizontal direction of the first surface 211a is not particularly limited and only has to be width that can stably carry the cell aggregate C. Such width is, for example, 0.1 to 0.5 mm. In this embodiment, the width d1 in the horizontal direction of the first surface 211 a is 0.3 mm.

The side section 220 includes the second surface 221, which is the curved surface having the second curvature, and includes the lower end 220d smoothly connected to the bottom section 210a via the continuous section 230 and the upper end 220u including the peripheral edge. The second curvature is larger than the curvature of the first surface 211a (in this embodiment, zero). Depending on the size of the recessed section 200a, for example, when a maximum diameter of an opening of the recessed section 200a formed on the upper surface 110 side is 0.5 mm, such a curvature exceeds zero and is equal to or smaller than 20 (mm⁻¹) and is desirably equal to or larger than 6.66 (mm⁻¹) and equal to or smaller than 20 (mm⁻¹). In this case, the curvature radius r2 is equal to or larger than 0.05 mm and equal to or smaller than 0.15 mm. In this embodiment, in the recessed section 200a, the maximum diameter of the opening of which is 0.37 mm, the side section 220 is illustrated in which the second surface 221, the second curvature of which is 7.69 (mm⁻¹) and the curvature radius r2 of which is 0.13 mm, is formed. The position and the vertical height of the upper end of the second surface 221 and the shape and the like of the side section 220 are the same as those explained above in the first embodiment. Therefore, explanation thereof is omitted.

As explained above, with the well plate 100a of this embodiment, the curvature of the first surface 211 a is zero and the bottom section 210a is the flat surface. Therefore, the cell aggregate C is less easily deformed and more stably carried on the bottom section 210a having the flat surface. The cell aggregate C carried on such a flat bottom section 210a is easily observed by an image pickup device such as a phase contrast microscope provided outside.

### (Third Embodiment)

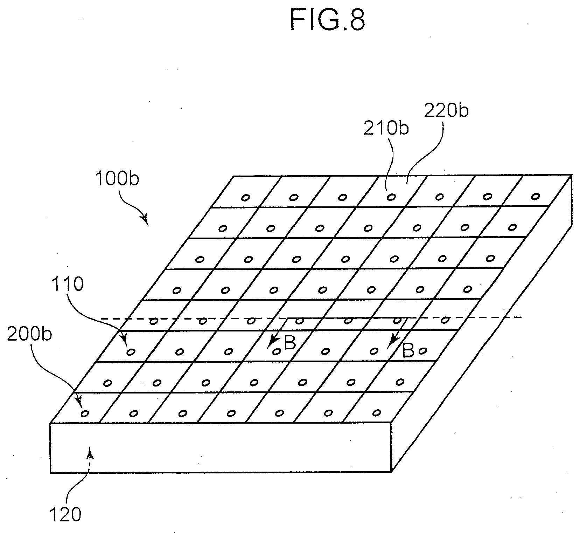

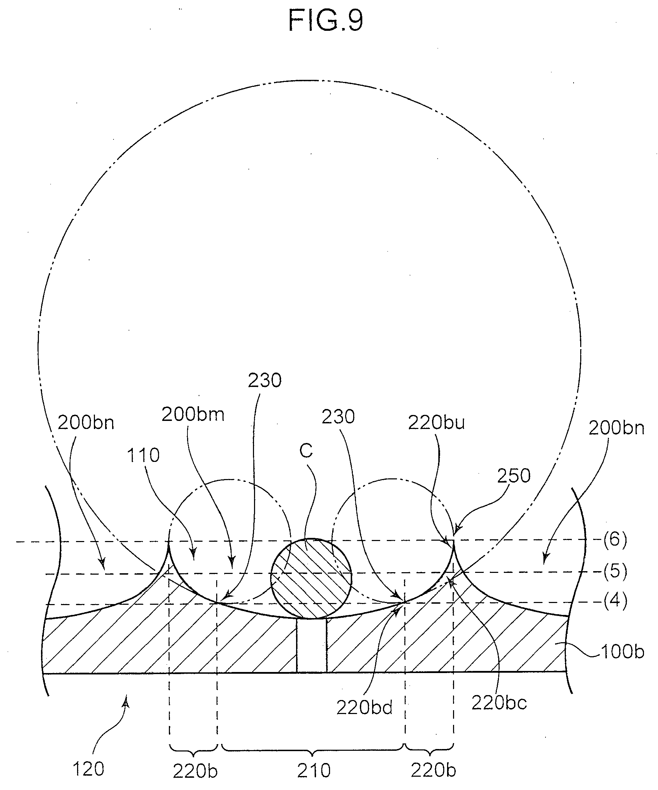

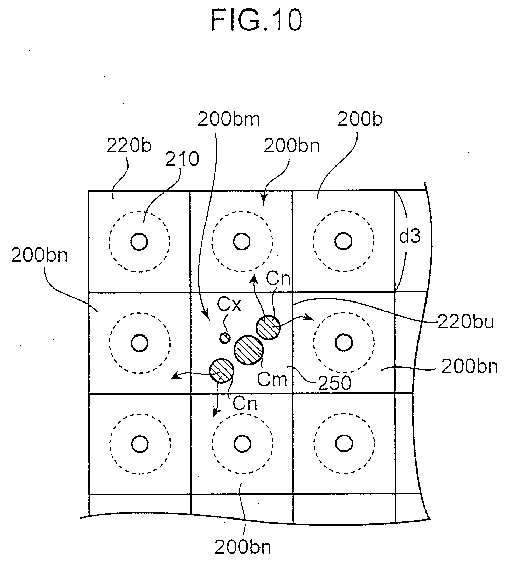

A well plate of a third embodiment of the present invention is explained in detail with reference to the drawings. Fig. 8 is a perspective view of a well plate 100b of this embodiment. Fig. 9 is a sectional view of a recessed section 200b of this embodiment and is a sectional view in a position indicated by B-B in Fig. 8. Fig. 10 is a top view of the recessed section 200b of this embodiment. The well plate 100b of this embodiment has a configuration same as the configuration of the well plate 100 (see Fig. 1 and Fig. 2) explained above in the first embodiment except that the shape of a side section 220b of the recessed section 200b is different and, therefore, the upper surface shape of the recessed section 200b is different. Therefore, redundant components are denoted by the same reference numerals and signs and explanation of the components is omitted as appropriate.

In the well plate 100b of this embodiment, a plurality of recessed sections 200b having a shape recessed from the upper surface 110 side to the lower surface 120 side are formed in a carrying position of the cell aggregate C. The shape of an opening in top view in each of the plurality of recessed sections 200b is a square (a quadrangle). The plurality of recessed sections 200b are arrayed in a matrix shape. The plurality of recessed sections 200b include peripheral edges at upper ends 220bu of the side sections 220b. The peripheral edge included in each of the plurality of recessed sections 200b is connected to the respective peripheral edges of the other recessed sections 200b adjacent in four directions in top view. A pointed peak section 250b is formed. Therefore, for example, even when a plurality of cell aggregates and impurities are carried on one recessed section 200b, the plurality of cell aggregates are easily separated by applying external force such as vibration. More specifically, as shown in Fig. 10, for example, when three cell aggregates (the cell aggregate Cm and the cell aggregates Cn) and the impurity Cx are carried on a recessed section 200bm, the cell aggregates Cn and the impurity Cx are easily separated from the recessed section 200bm to recessed sections 200bn in four directions adjacent to the recessed section 200bn by applying the external force such as the vibration. As a result, only the cell aggregate Cm is easily carried on the recessed section 200bm. For example, when the cell aggregate Cm carried on the recessed section 200bm is sucked by a suction pipet attached with a suction chip, as explained above with reference to Fig. 5 in the first embodiment, the cell aggregate Cm is not sucked together with the cell aggregates Cn carried on the neighboring recessed sections 200n. Further, since the respective plurality of recessed sections 200b are densely arrayed in the matrix shape, the number of recessed sections 200b that can be formed in one well plate 100b increases. Area efficiency is high. Length d3 of one side of the peak section 250b partitioning adjacent recessed sections (e.g., the recessed section 200bm and the recessed sections 200bn) is larger than length d2 (see Fig. 3) of one side of the peak section 250 explained above in the first embodiment. Therefore, for example, a cell aggregate dropped to the upper surface 110 of the well plate 100b from above the well plate 100b is easily urged to drop to the recessed section 200b when the cell aggregate approaches such a peak section 250b.

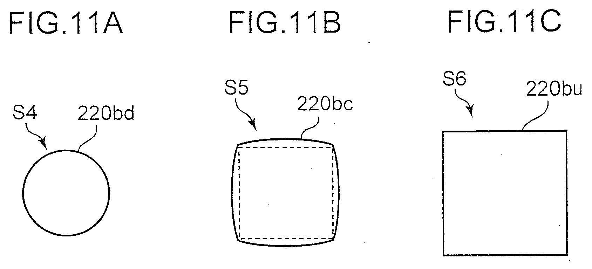

Note that, as explained above, the shape of the opening in top view in each of the plurality of recessed sections 200b of this embodiment is the square. That is, the shape of the upper end 220bu of the side section 220b is the square in top view. On the other hand, since a lower end 220bd of the side section 220b is smoothly connected to the bottom section 210 by the continuous section 230 explained above in the first embodiment, the shape of the lower end 220bd is the same as the outer peripheral shape (e.g., a circular shape) of the bottom section 210. Therefore, the shape of the side section 220b on the lower end 220bd side is the same as the outer peripheral shape of the bottom section 210 in top view. The shape of the side section 220b on the upper end 220bu side has a shape continuously deformed from the lower end 220bd side to the upper end 220bu side to be a square in top view. This is more specifically explained with reference to Fig. 11. Fig. 11 is a schematic view of end face shapes of the side section 220b in cross sectional positions (4) to (6) in Fig. 9. Fig. 11A is a schematic view of the end face shape of the side section 220b in the cross sectional position (4) in Fig. 9. Fig. 11B is a schematic view of the end face shape of the side section 220b in the cross sectional position (5) in Fig. 9. Fig. 11C is a schematic view of the end face shape of the side section 220b in the cross sectional position (6) in Fig. 9. As shown in Fig. 11A, in Fig. 9, a shape S4 of the lower end 220bd of the side section 220b in the cross sectional position (4) is the same as the outer peripheral shape of the bottom section 210. In this embodiment, it is illustrated that the outer peripheral shape of the bottom section 210 is a circular shape and the end face shape of the lower end 220bd of the side section 220b is also the circular shape. On the other hand, as shown in Fig. 11B, in Fig. 9, the shape of a center section 220bc of the side section 220b in the cross sectional position (5) is a shape similar to the square. However, sides connecting vertexes are deformed into a slightly rounded shape. As shown in Fig. 11C, in Fig. 9, the shape of the upper end 220bu of the side section 220b in the cross sectional position (6) is a square. In this way, the shape of the side section 220b is continuously deformed from the lower end 220bd side to the upper end 220bu side in top view. Therefore, when the cell aggregate C moves from the bottom section 210 climbing over the side section 220b when external energy such as vibration is applied thereto (in particular, near the continuous section 230 where the cell aggregate C approaches the side section 220b from the bottom section 210), internal energy is less easily lost and the cell aggregate C is easily separated climbing over the side section 220b.

As explained above, with the well plate 100b of this embodiment, the cell aggregate C is carried on the bottom section 210 and the shape of the cell aggregate C is not excessively deformed. As explained above with reference to Fig. 10, if the plurality of cell aggregates (the cell aggregate Cm and the cell aggregates Cn) are carried on the bottom section 210m or the impurity Cx is carried on the bottom section 210m, by applying the external force such as the vibration, the cell aggregates Cn are easily separated from the recessed section 200m to the recessed sections 200n. As a result, only the cell aggregate C is carried on the recessed section 200m. With the well plate 100 of this embodiment, when the cell aggregate Cm to be sucked carried on the recessed section 200m is sucked, even when the cell aggregates Cn are carried on the neighboring recessed sections 200n, the cell aggregates Cn are not simultaneously sucked. Only the cell aggregate Cm to be sucked can be easily sucked and selected. Further, since the respective plurality of recessed sections 200b are arrayed in the matrix shape, the number of recessed sections 200b that can be formed per one well plate 100b increases. Area efficiency is high.

### (Fourth Embodiment)

### <Subject selection device>

A subject selection device including a well plate of the present invention is explained in detail with reference to the drawings. In this embodiment, as an example, a subject selection device including the well plate 100 (see Fig. 1) explained above in the first embodiment is explained.

Fig. 12 is a schematic view for explaining the configuration of a subject selection device 300 of this embodiment. The subject selection device 300 includes the well plate 100 including the recessed section 200 that carries the cell aggregate C to be sucked (see Fig. 3), a petri dish 310 (container) including an inner bottom section 311 and opened in an upper part, a stage 320 on which the petri dish 310 is placed, a vibration generating device 330 (vibration generating means) that applies vibration to the well plate 100 retained in the petri dish 310 placed on the stage 320, a condenser 340 (irradiating means) arranged to be separated upward from the well plate 100 placed on the stage 320 to irradiate, from above, irradiation light on the cell aggregate C carried on the recessed section 200, the image pickup device 350 (observing means) disposed below the well plate 100 placed on the stage 320 to observe, from below, the cell aggregate C carried on the recessed section 200, a display device 351 attached to the image pickup device 350, a suction chip 360 for sucking the cell aggregate C carried on the recessed section 200, a suction pipet 370 that generates a suction force for the suction, and a moving device 380 (driving means) for moving the suction pipet 370 up and down. The cell culture solution Lm1 is stored in the petri dish 310. The well plate 100 is immersed in the cell culture solution Lm1 in the petri dish 310. The lower surface of the well plate 100 is separated from the inner bottom section 311 of the petri dish 310 via a spacer 130. Note that the condenser 340 and the image pickup device 350 are respectively devices configuring an illumination system and an image pickup system of an inverted phase contrast microscope.

The stage 320 is a horizontal flat tabular stand including a circular holder (not shown in the figure) that holds the petri dish 310. A position adjusting mechanism (not shown in the figure) for manually or automatically moving the well plate 100 to the front and the back and the left and the right is provided in the stage 320. The position of the well plate 100 placed on the stage 320 is adjusted by the position adjusting mechanism such that the condenser 340 is disposed above the recessed section 200 in which the cell aggregate C to be sucked is carried and the image pickup device 350 is disposed below the recessed section 200. Consequently, irradiation light from a light source of the condenser 340 is irradiated from above the recessed section 200, which carries the cell aggregate C to be sucked, and made incident on the image pickup device 350 below the recessed section 200.

The condenser 340 is disposed to be separated from the well plate 100 above the well plate 100 placed on the stage 320 and provided in order to irradiate, from above, the irradiation light on the cell aggregate C carried on the recessed section 200. The condenser 340 includes a substantially cylindrical housing and includes, in the housing, a light source (a halogen lamp (6V, 30W)), a collector lens, a ring slit, an aperture top, and a condenser lens not shown in the figure. The light source is not particularly limited. Besides the halogen lamp, for example, a tungsten lamp, a mercury lamp, a Xenon lamp, and a light emitting diode (LED) can be used. The ring slit is a light blocking plate having annular holes opened therein and is built in the position of the aperture stop of the condenser 340. The irradiation light irradiated from the light source in the condenser 340 passes through the collector lens, the holes of the ring slit, the aperture stop, and the condenser lens and is irradiated on the cell aggregate C carried on the recessed section 200 and thereafter made incident on the image pickup device 350.

The image pickup device 350 is disposed below the petri dish 310 placed on the stage 320 and is provided in order to observe, from below, the cell aggregate C carried on the recessed section 200. The image pickup device 350 includes a not-shown objective lens for phase difference, an aperture (a lens optical system) of the objective lens, a phase plate, a field diaphragm of an eyepiece, the eye piece, a CCD (Charge Coupled Device) image sensor, which is an image pickup device, and an image processing section that are not shown in the figure, and the display device 351. The phase plate is a ring-like semitransparent tabular body. The phase plate attenuates the intensity of light passing through the phase plate and delays a phase by 1/4. The CCD image sensor converts an optical image formed on a light receiving surface into an electric image data signal. The image processing section applies image processing such as gamma correction and shading correction to image data according to necessity. The display device 351 displays the image data after the image processing. A user observes an image displayed on the display device 351. The irradiation light diffracted by the cell aggregate C is made incident on the objective lens for phase difference and focused. At this point, since most of the irradiation light passes through parts other than the phase plate, the phase of the irradiation light remains delayed by 1/4 wavelength. The direct light and the diffracted light have the same phase and intensify each other through interference. As a result, the cell aggregate C is observed brightly.

In this embodiment, the disposition of the components of the condenser 340 and the image pickup device 350 is adjusted to be a Koehler illumination system. That is, concerning the radiation light, the light source, the aperture stop, and the exit aperture of the objective lens are disposed at a conjugate point. Concerning an image of a specimen, the field diaphragm, the cell aggregate C (a sample), the field diaphragm of the eyepiece, the light receiving surface of the CCD image sensor are disposed to be a conjugate point. The Koehler illumination system forms an image of the light source in an aperture stop position and forms an image of the field diaphragm on a specimen surface to thereby brightly illuminate the cell aggregate C, which is the specimen, without unevenness. Since the field diaphragm and the aperture stop can be caused to independently function, it is possible to adjust an amount and a range of light on the specimen surface.

The suction pipet 370 is a tubular member that can generate a suction force. The suction chip 360 for sucking the cell aggregate C carried on the recessed section 200 is connected to the suction pipet 370. When the suction pipet 370 generates a suction force, a suction force is generated in a tubular passage 360p of the suction chip 360. The cell aggregate C is sucked from the suction port and collected. The suction pipet 370 is connected to the moving device 380 and used. Driving of the suction pipet 370 is controlled by the moving device 380. The suction pipet 370 is moved up and down.

The suction chip 360 has a shape bent in an L shape. The suction chip 360 is connected to the suction pipet 370 such that a distal end portion 361 is in the substantially vertical direction and a rear end portion 362 takes a lateral posture extending in the lateral direction. Therefore, when the disposition of the components of the condenser 340 and the image pickup device 350 are adjusted to be the Koehler illumination system, it is possible to dispose the distal end portion 361 between the condenser 340 and the well plate 100 while maintaining the disposition of the components. As a result, it is possible to dispose the moving device 380 obliquely above the well plate 100 and dispose the distal end portion 361 in a gap between the condenser 340 and the well plate 100, without disposing the moving device 380 in a position where the moving device 380 blocks the irradiation light from the condenser 340. Note that a method of causing the distal end portion 361 to enter the gap between the condenser 340 and the well plate 100 is not particularly limited. For example, it is possible to adopt a method of moving the stage 320 in the front-back direction and the left-right direction.

The suction pipet 370 can be connected to the moving device 380 in a lateral posture. The moving device 380 is a device for moving the connected suction pipet 370 up and down in a state in which the lateral posture is maintained. The moving device 380 is disposed obliquely above the stage 320. The moving device 380 includes a main body section 381, to which the suction pipet 370 is connected, and a guide section 382 in which the main body section 381 travels. The main body section 381 includes, in a substantially rectangular parallelepiped housing, a motor (not shown in the figure) that moves the main body section 381 in the up-down direction to thereby move the suction pipet 370 up and down, a controller (not shown in the figure) that controls the motor, and a syringe pump (not shown in the figure) that generates a suction force in the suction pipet 370. On the outer side of the housing of the main body section 381, a suction port in which a suction force is generated by the syringe pump, that is, a connection port (not shown in the figure) connected to the suction pipet 370 is formed. A linear gear (a rack) is provided in the guide section 382. A circular gear (a pinion) is provided in the main body section 381. The motor controlled by the controller is driven, whereby the main body section 381 travels in the guide section 382. Note that, besides moving the main body section 381 up and down, the motor can also move the main body section 381 in the front-back direction and the left-right direction such that the suction port of the suction chip 360 is captured in a depth of field of the objective lens of the image pickup device 350 and perform calibration of the suction device. The calibration is performed as appropriate, for example, during replacement of the suction chip 360 and during a device start.

A method of selecting the cell aggregate C using the subject selection device 300 of this embodiment is specifically explained.

First, a cell culture solution Lm2 including, for example, the cell aggregate C and the impurity Cx (see Fig. 3) is dripped from above the well plate 100. The drip of the cell culture solution Lm2 can be performed by a suction pipet Pa attached with the suction chip Ta. The cell aggregate C and the impurity Cx included in the dripped cell culture solution Lm2 are carried on the recessed section 200 of the well plate 100. The cell aggregate C and the impurity Cx carried in the carrying position of the recessed section 200 are observed by the image pickup device 350 and displayed on the display device 351.

At this point, as shown in Fig. 3, a plurality of cell aggregates (the cell aggregate Cm and the cell aggregates Cn) and the impurity Cx are sometimes carried on one recessed section 200m. Therefore, in such a case, the subject selection device 300 of this embodiment generates vibration with the vibration generating device 330, which applies vibration to the well plate 100 retained in the petri dish 310 placed on the stage 320, and separates the cell aggregates Cn and the impurity Cx such that only one cell aggregate Cm is carried on the recessed section 200m. Specifically, as explained above with reference to Fig. 4 in the first embodiment, when the vibration is applied to the well plate 100 by the vibration generating device 330, the cell aggregates Cn is separated to the outside of the recessed section 200m (e.g., to the neighboring recessed sections 200n) from the bottom section 210m through the side section 220m. Note that the impurity Cx drops to the inner bottom section 311 of the dish from the through-hole 240 of the recessed section 200m. As a result, only one cell aggregate Cm is carried on the recessed section 200m.

When it is confirmed by the image pickup device 350 and the display device 351 attached to the image pickup device 350 that one cell aggregate C (cell aggregate Cm) is carried on the recessed section 200, the cell aggregate C is sucked by the suction pipet 370 including the suction chip 360. Specifically, the suction chip 360 is inserted in the downward direction into the recessed section 200, in which the cell aggregate C is carried, according to the movement in the downward direction of the main body section 381. The suction port 363 is brought close to the cell aggregate C. The position of the cell aggregate C and the position of the suction port 363 are displayed on the display device 351 of the image pickup device 350. Therefore, the suction port 363 is accurately brought close to the cell aggregate C while the position of the suction port 363 is checked. The irradiation light from the condenser 340 is not blocked by parts other than the distal end portion 361. Therefore, the cell aggregate C is observed under sufficient irradiation light.

Thereafter, the subject selection device 300 drives the syringe pump (not shown in the figure) of the moving device 380, generates a suction force in the tubular passage 360p of the suction chip 360, and sucks the cell aggregate C from the suction port 363. At this point, as explained above with reference to Fig. 5 in the first embodiment, in some case, the cell aggregates Cn are also carried on the recessed sections 200n adjacent to the recessed section 200m in which the cell aggregate Cm to be sucked is carried. However, even if the liquid flow A1 is generated in order to suck the cell aggregate Cm, the cell aggregates Cn in the recessed sections 200n are not simultaneously sucked. Only the cell aggregate Cm to be sucked is sucked. Since a state of the recessed section 200m (presence or absence of the cell aggregate Cm) is displayed on the display device 351, it is possible to easily distinguish success or failure of the collection.

Finally, the subject selection device 300 moves the main body section 381 in the upward direction and lifts the distal end portion 361 from the recessed section 200. The subject selection device 300 discharges the sucked cell aggregate C (cell aggregate Cm) to a plate for collection (not shown in the figure) adjacent to the well plate 100 on the stage 320 on which the well plate 100 is placed and completes the selection.

As explained above, with the subject selection device 300 of this embodiment, it is possible to apply, with the vibration generating device 330, vibration to the well plate 100 in the petri dish 310 placed on the stage 320. Even if a plurality of cell aggregates C are carried on the bottom section 210 of the recessed section 200 of the well plate 100 or the impurity Cx other than the cell aggregate C is carried on the bottom section 210, it is possible to separate the plurality of cell aggregates C or the impurity Cx such that only one cell aggregate C is carried in the carrying position of the recessed section 200. Further, when one cell aggregate C carried on the recessed section 200 is sucked by the suction pipet 370 attached with the suction chip 360, even if the cell aggregate C is carried on the neighboring recessed section 200, the cell aggregate C in the neighboring recessed section 200 is not simultaneously sucked even if a liquid flow is generated in order to suck the cell aggregate C. Only the cell aggregate C to be sucked is sucked. As a result, with the subject selection device 300 of this embodiment, it is possible to appropriately select only target one cell aggregate C.

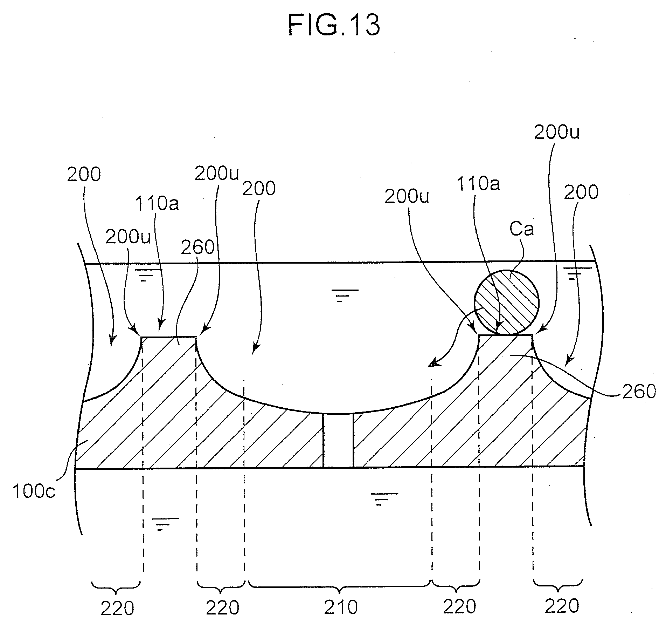

The embodiments of the present invention are explained above. However, the present invention is not limited to the embodiments. For example, modified embodiments explained below can be adopted.
(1) In the embodiments, the cell aggregate is illustrated as the subject carried on the recessed section. In the present invention, instead of the cell aggregate, a tablet, a capsule, a granulated granule, and the like and a cell deriving from an organism used in the fields of biotechnology-related techniques and medicines may be used as the subject.
   Note that, in the present invention, it is desirable to use the cell deriving from the organism as the subject and it is more desirable to use a cell aggregate deriving from an organism as the subject. That is, a cell or the like carried on a conventional well plate (platen) is easily deformed along the shape of a through-hole and characteristics of the cell or the like sometimes change. The carried cell sometimes firmly fits in the through-hole. When the cell is forcibly collected by suction or the like, the cell is sometimes damaged. Further, a plurality of cells sometimes fit in one through-hole. In such a case, for example, even if external force such as vibration is applied to the cells, the cells are not easily separated. It is difficult to appropriately collect one cell. However, with the well plate of the present invention, the carried cell is less easily deformed. Even if a plurality of cells are carried, the plurality of cells can be easily separated by applying external force such as vibration. It is possible to collect the cell without changing characteristics of the cell or damaging the cell. As a result, the cell is accurately measured. Results with high reliability are obtained in various experiments and the like.
   In the cell aggregate deriving from the organism, a biological similar environment that takes into account mutual action among cells is reconstructed on the inside of the cell aggregate. Therefore, a result that takes into account functions of the respective cells compared with a test result obtained using one cell is obtained. Experiment conditions can be aligned with conditions more conforming to an environment in an organism. Therefore, the cell aggregate deriving from the organism is considered important in a regenerative medicine field and a development field of drugs such as an anti-cancer drug. Specific examples of the cell aggregate include BxPC-3 (human pancreatic adenocarcinoma cells), an embryonic stem cell (an ES cell), and an induced pluriopotent stem cell (an iPS cell). In general, such a cell aggregate is formed by several to several hundred thousand aggregated cells. With the well plate of the present invention, the carried cell aggregate is less easily deformed. Even if a plurality of cell aggregates are carried, it is possible to easily separate the plurality of cell aggregates by applying external force such as vibration thereto. It is possible to collect the cell aggregate without changing characteristics of the cell aggregate or damaging the cell aggregate. As a result, since the cell aggregate is accurately measured, it is possible to obtain a result with high reliability in the fields of biotechnology-related techniques and medicines (including the regenerative medicine field and the development field of drugs such as the anti-cancer drug).
(2) In the embodiments, the cell aggregate is retained in the cell culture solution. Instead, in the well plate of the present invention, liquid that does not deteriorate the characteristics of the cell aggregate can be used as appropriate as the liquid for retaining the cell aggregate. Examples of representative liquid include, besides media such as a basal medium, a synthetic medium, an Eagle's medium, an RPMI medium, a Fischer's medium, a Ham's medium, an MCDB medium, and blood serum, a cell freezing solution such as glycerol and Cellbanker (manufactured by Juji Field Inc.), formalin, a reagent for fluorescent dying, an antibody, refined water, and saline. A culture preservation solution adapted to the cell aggregate can be used. For example, when the cell aggregate is BxPC-3 (human pancreatic adenocarcinoma cell), a culture preservation solution obtained by adding, according to necessity, a supplement such as antibiotic or sodium pyruvate to an RPMI-1640 medium mixed with 10% of fetal bovine serum (FBS) can be used.
(3) In the embodiments, the recessed section in which the through-hole is formed in the bottom section is illustrated. Instead, in the well plate of the present invention, the through-hole is not essential. That is, even when impurities having a diameter smaller than the cell aggregate are included in the cell culture solution including the cell aggregate, the impurities can be removed in advance by performing treatment such as filtering using a filter.
(4) In the embodiments, it is illustrated that each of the plurality of recessed sections is connected to the peripheral edge of the upper end of the side section and the peak section is formed. Instead, in the well plate of the present invention, the peak section is not essential. Fig. 13 is a sectional view for explaining a well plate 100c of a modification of the first embodiment. Note that a recessed section of the well plate 100c has a configuration same as the configuration of the recessed section 200 explained above in the first embodiment. Therefore, the recessed section is denoted by the same reference numeral and explanation of the recessed section is omitted as appropriate. In the well plate 100c of this modification, the peripheral edges of the upper ends 220u of the side sections 220 of the recessed sections 200 adjacent to each other are not connected. A wall section 260 that partitions the adjacent recessed sections 200 is formed. In this case, for example, the cell aggregate C is sometimes temporarily retained on an upper surface 110a of the wall section 260. A cell aggregate Ca indicates the cell aggregate retained on the upper surface 110a of the wall section 260. However, such a cell aggregate Ca can be easily dropped to the bottom section 210 of the recessed section 200 by applying external force such as vibration thereto.
(5) In the embodiments, the upper end of the side section of each of the plurality of recessed section is connected to the upper ends of the side sections of the recessed sections adjacent thereto. As explained with reference to Fig. 6A to Fig. 6C and Fig. 11A to Fig. 11C, it is illustrated that the shape of the side section is continuously deformed from the lower end side to the upper end side and the shape of the opening of the recessed section in top view is formed in the regular hexagon or the square at the upper end. In the well plate of the present invention, instead, the shape in top view at the upper end does not need to be formed in the regular hexagon or the square and may be the rounded shape similar to the regular hexagon explained with reference to Fig. 6B or may be the rounded shape similar to the square explained with reference to Fig. 11B.
(6) In the embodiments, the recessed section is illustrated in which the first surface having the zero curvature or the first curvature is formed in the entire region of the bottom section and the second surface having the second curvature is formed in the entire region of the side section. Instead, in the present invention, the first surface having the zero curvature or the first curvature may formed in at least a part of the bottom section and the second surface having the second curvature may be formed in at least a part of the side section.
(7) In the embodiments (the fourth embodiment), it is illustrated that the subject selection device includes the vibration generating device as the device for applying external force to the well plate. Instead, in the present invention, an inclining device (e.g., a rocking mixer RM-80, manufactured by AS ONE Corporation) may be included as the device for applying external force to the well plate. With the inclining device, by inclining the well plate, it is possible to separate and disperse the cell aggregate carried on the recessed section.

Inventions including configurations explained below are mainly included in the specific embodiments explained above.

A well plate according to an aspect of the present invention is a well plate that carries, in a carrying position, a subject retained in liquid, the well plate including an upper surface, a lower surface, and a plurality of recessed sections formed in the carrying position, wherein each of the plurality of recessed sections is opened on the upper surface side, has a shape recessed from the upper surface side to the lower surface side, and includes, in a cross section in an up-down direction, a bottom section in which a first surface having a zero curvature or a first curvature is formed and the subject is carried, and a side section in which a second surface having a second curvature larger than the first curvature and continuous to the first surface is formed.

In this way, the well plate of the present invention includes the plurality of recessed sections formed in the carrying position. Each of the plurality of recessed sections is opened on the upper surface side, has a shape recessed from the upper surface side to the lower surface side, and includes, in the cross section in the up-down direction, the bottom section in which the first surface having the zero curvature or the first curvature is formed and the subject is carried. The recessed section includes the side section in which the second surface having the second curvature larger than the first curvature and continuous to the first surface is formed. Therefore, the bottom section has the zero curvature or the first curvature smaller than the second curvature and is relatively flat. Therefore, the subject is carried on such a relatively flat bottom section and the shape of the subject is not excessively deformed. If a plurality of subjects are carried on the bottom section or impurities other than the subject are carried on the bottom section, by applying external force such as vibration, the subject is easily separated to the outside of the original recessed section by stress applied by the vibration from the bottom section, in which the first surface having the first curvature is formed, through the side section, in which the second surface having the larger second curvature is formed. As a result, one subject is carried on the original recessed section. Since the second curvature of the second surface is larger than the first curvature of the first surface, even if a slight liquid flow occurs around the subject carried on the bottom section, the subject does not move climbing over the side section with the liquid flow. Specifically, for example, even if a liquid flow occurs around the subject carried on the bottom section when the suction port of the suction chip of the suction pipet is brought close to the subject during suction, the subject is not moved to climb over the side section formed on the second surface by the liquid flow of this degree. Similarly, for example, when a plurality of recessed sections are adjacent to one another and subjects are carried on bottom sections of the respective recessed sections, even if a suction force for sucking the subject carried on one recessed section with the suction pipet is generated, the subjects carried on the other recessed sections are less easily affected by a liquid flow caused by a suction operation. That is, when the subject carried on one recessed section is sucked, the subjects carried on the neighboring recessed sections are not simultaneously sucked. Therefore, only one subject is easily collected.

In the configuration, it is preferable that the side section of each of the plurality of recessed sections includes a peripheral edge at an upper end thereof, the plurality of recessed sections include a first recessed section and a second recessed section adjacent to the first recessed section, and the peripheral edges of the first recessed section and the second recessed section are connected to each other.

With such a configuration, the peripheral edges of the first recessed section and the second recessed section are connected to each other. Therefore, even if a plurality of subjects are carried on the bottom section of the first recess or impurities other than the subject are carried on the bottom section, for example, by applying external force such as vibration, the subject comes off the carrying position of the first recessed section and is easily separated to the second recessed section. Specifically, when the peripheral edges of the first recessed section and the second recessed section are connected to each other, a peak section that partitions the first recessed section and the second recessed section is formed between the first recessed section and the second recessed section. Since the peak section has a relatively pointed shape, if the subject coming off the carrying position of the first recessed section according to the application of the external force such as the vibration only slightly climbs over the peak section, thereafter, the subject easily drops to the carrying section along the side surface of the second recessed section. As a result, the subject is easily separated from the first recessed section to the second recessed section.

In the configuration explained above, it is preferable that the side section of each of the plurality of recessed sections includes a peripheral edge at an upper end thereof, a shape of the opening in top view in each of the plurality of recessed sections is a quadrangle, and the plurality of recessed sections are arrayed in a matrix shape and the peripheral edges of the plurality of recessed sections are connected.

With such a configuration, the shape of the opening in top view in each of the plurality of recessed sections is a quadrangle, the plurality of recessed sections are arrayed in the matrix shape, and the peripheral edges are connected. Therefore, even if a plurality of subjects are carried on the bottom section of one recessed section or impurities other than the subject are carried on the bottom section, for example, by applying external force such as vibration, the subject comes of the original carrying position and is easily separated to the other recessed sections adjacent in four directions. The number of recessed sections that can be formed per one well plate is large. Area efficiency is high.

In the configuration explained above, it is preferable that the side section of each of the plurality of recessed sections includes a peripheral edge at an upper end thereof, a shape of the opening in top view in each of the plurality of recessed sections is a regular hexagon, and the plurality of recessed sections are arrayed in a honeycomb shape and the peripheral edges of the plurality of recessed sections are connected.

With such a configuration, the shape of the opening in top view in each of the plurality of recessed sections is the regular hexagon, and the plurality of recessed sections are arrayed in the honeycomb shape and the peripheral edges are connected. Therefore, even if a plurality of subjects are carried on the bottom section of one recessed section or impurities other than the subject are carried on the bottom section, for example, by applying external force such as vibration, the subject comes of the original carrying position and is easily separated to the other recessed sections adjacent in six directions. Since the shape in top view of the recessed section is the regular hexagon and is a shape relatively close to a circular shape, the subject less easily adheres to the side section of the recessed section and is less easily deformed. The number of recessed sections that can be formed per one well plate is large. Area efficiency is high.

In the configuration explained above, it is preferable that through-holes having a diameter smaller than a diameter of the subject are formed from the upper surface side to the lower surface side in the bottom sections of the plurality of recessed sections.

With such a configuration, the through-holes having the diameter smaller than the diameter of the subject are formed from the upper surface side to the lower surface side in the bottom sections of the plurality of recessed sections. Therefore, when impurities having a diameter smaller than the through-holes are included, the impurities drop from the through-holes and removed. Since the diameter of the through-holes is smaller than the subject, the subject less easily fits in the through-holes and is less easily deformed.

In the configuration explained above, it is preferable that the subject is a cell deriving from an organism.

With this configuration, the subject is the cell deriving from the organism. Therefore, the cell appropriately separated using the well plate of the present invention can be used in the fields of biotechnology-related techniques and medicines.

In the configuration explained above, it is preferable that the subject is a cell aggregate deriving from an organism.

In the cell aggregate deriving from the organism, a biological similar environment that takes into account mutual action among cells is reconstructed on the inside of the cell aggregate. Therefore, a result that takes into account functions of the respective cells compared with a test result obtained using one cell is obtained. Experiment conditions can be aligned with conditions more conforming to an environment in an organism. Therefore, the cell aggregate deriving from the organism is considered important in a regenerative medicine field and a development field of drugs such as an anti-cancer drug. Therefore, the cell aggregate appropriately separated using the well plate of the present invention can be used in the fields of biotechnology-related techniques and medicines (including the regenerative medicine field and the development field of drugs such as the anti-cancer drug).

A subject selection device according to another aspect of the present invention includes the well plate and vibration generating means for causing the well plate to vibrate.

With such a configuration, the subject selection device can apply vibration to the well plate with the vibration generating means. Even if a plurality of subjects are carried on the bottom section of the recessed section of the well plate or impurities other than the subject are carried on the bottom section, it is possible to separate the plurality of subjects or the impurities such that one subject is carried in the carrying position of the recessed section.

## Claims

1. A well plate that carries, in a carrying position, a subject retained in liquid, the well plate comprising an upper surface, a lower surface, and a plurality of recessed sections formed in the carrying position, wherein
each of the plurality of recessed sections is opened on the upper surface side, has a shape recessed from the upper surface side to the lower surface side, and includes, in a cross section in an up-down direction:
a bottom section in which a first surface having a zero curvature or a first curvature is formed and the subject is carried; and
a side section in which a second surface having a second curvature larger than the first curvature and continuous to the first surface is formed.

2. The well plate according to claim 1, wherein
the side section of each of the plurality of recessed sections includes a peripheral edge at an upper end thereof,
the plurality of recessed sections include a first recessed section and a second recessed section adjacent to the first recessed section, and
the peripheral edges of the first recessed section and the second recessed section are connected to each other.

3. The well plate according to claim 1, wherein
the side section of each of the plurality of recessed sections includes a peripheral edge at an upper end thereof,
a shape of the opening in top view in each of the plurality of recessed sections is a quadrangle, and
the plurality of recessed sections are arrayed in a matrix shape and the peripheral edges of the plurality of recessed sections are connected.

4. The well plate according to claim 1, wherein
the side section of each of the plurality of recessed sections includes a peripheral edge at an upper end thereof,
a shape of the opening in top view in each of the plurality of recessed sections is a regular hexagon, and
the plurality of recessed sections are arrayed in a honeycomb shape and the peripheral edges of the plurality of recessed sections are connected.

5. The well plate according to any one of claims 1 to 4, wherein a through-hole having a diameter smaller than a diameter of the subject is formed from the upper surface side to the lower surface side in the bottom section of each of the plurality of recessed section.

6. The well plate according to any one of claims 1 to 5, wherein the subject is a cell deriving from an organism.

7. The well plate according to claim 6, wherein the subject is a cell aggregate deriving from an organism.

8. A subject selection device comprising:
the well plate according to any one of claims 1 to 7; and
vibration generating means for causing the well plate to vibrate.
